# FASCICULE DE BREVET EUROPEEN

(11) **EP 0 839 537 B1**
(45) Date de publication et mention de la délivrance du brevet: **25.09.2002**
(21) Numéro de dépôt: 97402597.5
(22) Date de dépôt: 31.10.1997
(51) Int. Cl.: A61L 2/26, A61L 2/10

(54) **Dispositif de support d'instruments dans une enceinte notamment de décontamination. Enceinte correspondante**
Tragvorrichtung für chirurgische Instrumente in einem Sterilisationsraum und Raum dafür
Supporting appliance for surgical instruments in a sterilization enclosure and enclosure thereof

(30) Priorité: 04.11.1996 FR 9613399; 24.06.1997 FR 9707855
(43) Date de publication de la demande: 06.05.1998
(73) Titulaire: T.S.R. SA, 92110 Clichy (FR)
(72) Inventeur: Morello, Gérard, 78250 Hardricourt (FR)
(74) Mandataire: Robert, Jean-Pierre

(56) Documents cités:
- WO-A-93/17727
- WO-A-94/06478
- DE-C- 3 819 257
- GB-A- 2 090 141

## Description

La présente invention concerne une enceinte pour des instruments médicaux destinée à les décontaminer et à les maintenir décontaminés jusqu'à leur utilisation.

Il est connu de loger des instruments médicaux ou chirurgicaux dans une enceinte fermée à l'intérieur de laquelle on organise une atmosphère de décontamination soit au moyen d'un agent introduit dans cette enceinte, soit au moyen d'un rayonnement germicide émis dans l'enceinte, soit enfin par la combinaison des deux.

Dans le cas particulier d'instruments allongés, tels par exemple que des sondes d'échographie, ces instruments sont en une seule pièce avec des cordons de raccordement à des appareils extérieurs d'alimentation en énergie électrique et d'exploitation des signaux émis par la partie active de l'appareil. L'ensemble partie active et cordon de l'instrument peuvent être d'une longueur non négligeable.

Il a fallu trouver une manière de les loger facilement dans une enceinte de décontamination grâce à des moyens qui puissent prendre en compte la diversité de leur longueur, et la diversité de leur diamètre.

Pour résoudre ce problème on propose par la présente invention un dispositif de support particulièrement simple et avantageux grâce auquel la partie active des appareils est placée de manière optimale dans l'enceinte afin d'être le plus efficacement exposée aux agents germicides par contact ou par rayonnement tandis que la partie de cordon est disposée de manière à ne pas gêner le traitement de la partie active.

A cet effet, l'invention a donc pour objet une enceinte de décontamination, délimitée par un fond, au moins une paroi latérale et un couvercle supérieur, chaque instrument comportant une partie active et une partie de raccordement sous forme d'un câble, caractérisée en ce qu'elle comprend une potence s'étendant sensiblement parallèlement au fond à l'intérieur et en partie supérieure de l'enceinte, cette potence comportant une pluralité d'organes de suspension chacun d'eux étant destiné à coopérer avec une partie du câble voisine de la partie active de l'instrument.

Dans un mode particulier de réalisation, chaque organe de suspension comporte un doigt implanté dans une surface supérieure de la potence et une pince de suspension dont les deux branches sont réunies en une extrémité pourvue d'un orifice pour recevoir par enfichage le doigt susdit, les extrémités libres des branches de la pince étant conformées pour être resserrées sur la partie du câble de raccordement voisine de la partie active de l'instrument. La pince est alors mise en place sur l'instrument, plus exactement sur son câble, à l'extérieur de l'enceinte puis elle est enfichée par son extrémité percée sur l'un des doigts que comporte la potence. L'autre extrémité de chaque pince, qui enserre le câble de l'instrument, est située en porte-à-faux latéral de la potence si bien que les instruments sont écartés les uns des autres. La partie active de l'instrument pend alors à l'intérieur de l'enceinte où elle est exposée de manière optimale aux agents germicides tandis que le câble de raccordement est situé au-dessus de la pince pour pouvoir être pris en charge par un organe de support secondaire voisin de la paroi latérale de l'enceinte.

Les branches de cette pince peuvent être soumises à un effort élastique de rapprochement de manière à enserrer automatiquement le câble qui est placé entre elles. On peut également prévoir entre les branches un organe de manoeuvre qui assure leur rapprochement ou leur écartement de manière à pouvoir serrer positivement avec un effort contrôlé le câble entre elles et à libérer cet effort. Une vis de liaison des branches peut ainsi jouer ce rôle.

De manière simple, l'organe secondaire de support du câble de chaque instrument sera constitué par le bord supérieur lui-même de la paroi latérale de l'enceinte et, de préférence par le fond d'une échancrure ménagée dans la partie supérieure de ce dernier. Les câbles des instruments seront alors maintenus sensiblement horizontalement au voisinage du couvercle de l'enceinte pour ne pas encombrer inutilement l'espace de cette dernière où est logée la partie active de chaque instrument.

D'autres caractéristiques et avantages de l'invention ressortiront de la description d'un mode de réalisation donné ci-après à titre purement indicatif.

Il sera fait référence aux dessins annexés parmi lesquels :
- la figure 1 est une vue générale simplifiée d'une enceinte de décontamination équipée d'un dispositif de support conforme à l'invention,
- la figure 2 est une vue de détail de la pince mise en oeuvre dans le dispositif de support de la figure 1,
- la figure 3 est une vue générale semblable à la figure 1 d'une enceinte de décontamination mettant en oeuvre des sources de rayonnement germicide.

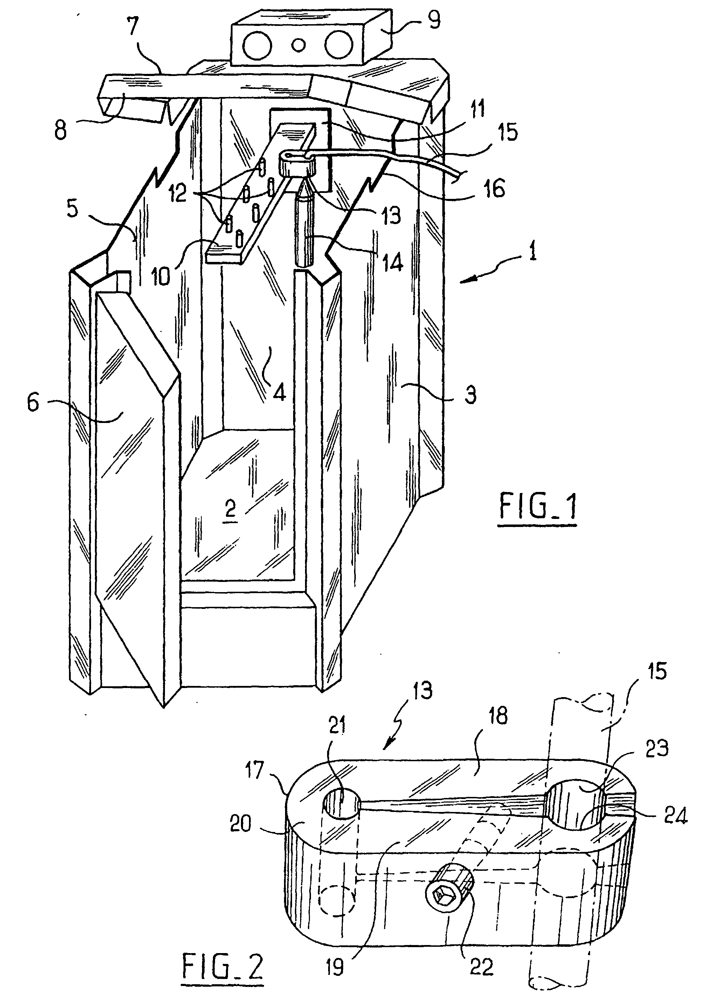

L'enceinte 1 représentée à la figure 1 comporte un fond 2, quatre parois latérales 3, 4, 5 et 6, la paroi latérale 6 formant une porte de façade ouvrante vers l'extérieur. Un couvercle 7 à bords tombants 8 permet d'accéder au volume intérieur de l'enceinte par le dessus et constitue un moyen simple de maintien de la porte 6 dans son état fermé. Cette enceinte possède un pupitre de commande 9.

La paroi latérale 4, opposée à la porte frontale 6, porte une potence 10 par l'intermédiaire d'une semelle 11. Cette potence est située en partie supérieure de l'enceinte et s'étend au-dessus du fond 2 en surplomb à l'intérieur de l'enceinte. Elle comporte une pluralité de doigts 12 qui forment des fiches verticales tournées vers le couvercle 7.

L'une de ces fiches, sur la figure, est équipée d'un organe 13 de suspension, décrit plus en détail en regard de la figure 2, pour un instrument 14 médical, chirurgical ou de diagnostic, par exemple une sonde d'échographie 14 qui est prolongée, de manière connue par un câble de raccordement 15.

Le câble 15 s'étend au-dessus de l'organe de suspension 13, sensiblement horizontalement en partie supérieure de l'enceinte jusqu'à une échancrure 16 ménagée dans la partie supérieure de la paroi latérale 3. Au-delà de cette échancrure, le câble sort de l'enceinte. On remarque sur la figure 1 que l'organe de suspension 13 est enfiché par l'une de ses extrémités sur un doigt 12 et est en porte-à-faux latéral de la potence 10 de sorte que chaque instrument 14 est suspendu écarté de la potence donc des autres instruments suspendus de l'autre côté de celle-ci.

Bien entendu, l'invention comprend également des variantes de réalisation non représentées. Par exemple, la potence 10 peut comporter des orifices pour l'enfichage de doigts qui eux seraient portés par chaque organe de suspension 13. Par exemple encore, la potence peut être latéralement équipée de clips élastiques entre les mâchoires de chacun desquels il serait possible d'engager le câble d'un instrument. On peut également prévoir des encoches latérales et fixer sur le câble des bagues annulaires qui formeraient butées de suspension de chaque instrument. Les encoches pourraient être en forme d'oeil fendu et les bagues épaulées pour qu'au moment de leur mise en place il se produise un verrouillage latéral interdisant à l'instrument suspendu d'échapper à l'encoche (par exemple lors de la mise en place d'un instrument adjacent).

On comprend de cette figure 1 que la partie câble de raccordement de chaque instrument est confinée en partie supérieure de l'enceinte dans une position sensiblement horizontale. Le couvercle possédant un jeu entre ses bords tombants 8 et l'extérieur de la paroi latérale, la partie des câbles extérieure à l'enceinte ne gêne pas la fermeture de l'enceinte. S'il y a besoin d'assurer une étanchéité, on aura prévu au niveau de l'encoche et de la zone correspondante du couvercle des moyens en correspondance (par exemple des joints déformables) pour l'assurer.

A la figure 2, l'organe 13 de suspension représenté affecte la forme d'une pince 17 avec deux branches 18 et 19 épaisses pour qu'à l'extrémité 20 où elles sont réunies, un orifice 21 d'un diamètre à peu près égal à celui d'une fiche 12 ait une longueur axiale suffisante pour une bonne fixation de la pince à la potence. Les deux branches 18 et 19 peuvent être rapprochées ou écartées l'une de l'autre par une vis 22 (sans qu'il y ait de variation sensible sur le diamètre de l'orifice 21) ces branches étant pourvues d'un évidement partiellement cylindrique 23, 24 pour épouser la surface externe d'un câble 15 et la serrer.

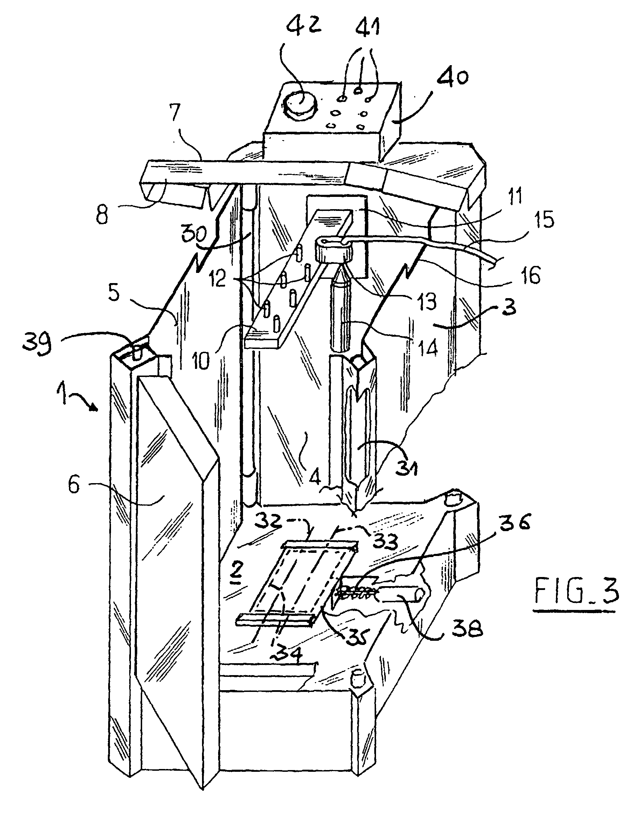

A la figure 3, on retrouve la plupart des éléments déjà décrits avec les mêmes références. L'enceinte ici représentée est adaptée à la décontamination par rayonnement. Pour ce faire elle est équipée de tubes émetteurs d'un rayonnement ultra-violet connus en eux-mêmes. Ces tubes sont, dans l'exemple représenté, au nombre de six. Quatre d'entre eux sont logés dans les angles de l'enceinte comme les tubes 30 et 31 tandis que les deux derniers 32, 33 sont logés au fond de l'enceinte sous la paroi de fond 2 pourvue d'une fenêtre 34 à l'aplomb de laquelle est située la potence 10.

Compte-tenu de la nature des instruments à décontaminer, on s'est rendu compte qu'il était extrêmement important de soumettre l'extrémité de leur partie active à un rayonnement direct. Comme les instruments sont suspendus, ladite extrémité faisant face au fond, les sources de rayonnement devaient se trouver en partie basse. Cette disposition présente cependant l'inconvénient d'exposer directement les tubes de fond à un risque de casse si l'un des instruments suspendus tombe. Ce risque n'est réel que lors de la mise en place ou du retrait des instruments car, une fois suspendus, les raisons de leur décrochement intempestif sont inexistantes.

C'est pourquoi la fenêtre 34 est munie d'un volet escamotable 35 (plein ajouré ou grillagé...) qui est rappelé dans sa position de couverture de cette fenêtre par un organe élastique de rappel (au moins un ressort 36). Par ailleurs le volet est attelé à un actionneur 38 (par exemple électromagnétique) qui, lorsqu'il est excité, déplace le volet 35 à l'encontre de l'effet du ressort de rappel de manière à découvrir la fenêtre 34. L'alimentation de l'actionneur 38 est réalisée en même temps que l'alimentation des tubes 30 à 33 lors de la fermeture du couvercle 7 qui agit sur un interrupteur 39 de fermeture du circuit électrique général. Le volet 35 peut être monté coulissant dans des guides latéraux au-dessus ou en-dessous de la paroi de fond 2 de l'enceinte, le ressort 36 et l'actionneur 38 pouvant également être situés sous cette paroi.

Sur le pupitre de commande 40 de l'appareil il Sur le pupitre de commande 40 de l'appareil il existe des voyants lumineux 41 qui constituent les témoins de fonctionnement de chacun des tubes radiants et de la position du volet 35. Un bouton 42 forme d'une part organe de commande marche-arrêt de l'appareil et d'autre part organe de réglage d'une temporisation pour ajuster le temps d'irradiation et d'excitation de l'actionneur 38. A la fin de la temporisation, les tubes s'éteignent et le volet 35 obture l'ouverture 34. Les tubes inférieurs sont ainsi protégés contre la chute d'une sonde au moment de leur mise en place dans l'enceinte (couvercle ouvert donc coupure de l'alimentation de l'actionneur) et au moment de leur extraction (le volet obturant la fenêtre depuis la fin de la temporisation).

Il faut noter enfin qu'il sera préférable de visualiser la position du volet 35 par un voyant 41 lié plutôt à un détecteur de sa position qu'à un détecteur de l'alimentation de son organe moteur pour être certain que l'irradiation n'est pas masquée par le volet resté en position d'obturation de la fenêtre malgré une alimentation correcte de son organe moteur.

## Revendications

1. Enceinte de décontamination pour instruments médicaux, délimitée par un fond (2), au moins une paroi latérale (3, 4, 5, 6) et un couvercle (7) supérieur, chaque instrument (14) comportant une partie active et une partie de raccordement sous forme d'un câble (15), **caractérisée en ce qu'**elle comprend une potence (10) s'étendant à l'intérieur et en partie supérieure de l'enceinte (1) parallèlement au fond (2) et en surplomb du fond (2), cette potence comportant une pluralité d'organes de suspension (12, 13) chacun d'eux étant destiné à coopérer avec une partie du câble (15) voisine de la partie active de l'instrument.

2. Enceinte selon la revendication 1, **caractérisé en ce que** chaque organe de suspension comporte un doigt (12) implanté dans une surface supérieure de la potence (10) et une pince (13) de suspension dont les deux branches (18, 19) sont réunies en une extrémité pourvue d'un orifice (21) pour recevoir par enfichage le doigt (12) susdit, les extrémités libres des branches de la pince étant conformées pour être resserrées sur la partie du câble (15) de raccordement voisine de la partie active de l'instrument.

3. Enceinte selon la revendication 2, **caractérisée en ce que** la pince comprend un organe (22) de manoeuvre des branches (18, 19) pour les rapprocher ou les éloigner l'une de l'autre.

4. Enceinte selon la revendication 2 ou la revendication 3, **caractérisée en ce qu'**elle comprend un organe (16) secondaire de suspension pour le câble de raccordement de chaque instrument solidaire de la paroi latérale de l'enceinte (3, 4, 5, 6) afin de maintenir ce dernier dans une position sensiblement horizontale entre la pince et cette paroi.

5. Enceinte selon la revendication 4, **caractérisée en ce que** l'organe secondaire de suspension est constitué par le bord supérieur de la paroi latérale (3) au fond d'une échancrure (16) de ce dernier.

6. Enceinte selon l'une quelconque des revendications précédentes, **caractérisée en ce qu'**elle comporte au moins un émetteur (32, 33) de rayonnement germicide ultraviolet situé dans une fenêtre (34) ménagée dans la paroi (2) de fond de l'enceinte sous la potence (10) et **en ce que** le fond (2) est équipé d'un volet (35) de protection de l'émetteur (32, 33), escamotable entre une première position dans laquelle il obture la fenêtre (34) et une seconde position dans laquelle il la découvre.

7. Enceinte selon la revendication 6, **caractérisée en ce que** le volet (35) est attelé à un organe de sa manoeuvre entre les deux positions susdites formé par un élément élastique (36) de rappel du volet (35) dans sa première position et par un élément moteur (38) qui, dans son état activé, développe un effort de déplacement du volet (35) vers sa deuxième position à l'encontre de l'effet de l'élément élastique de rappel (36).

8. Enceinte selon la revendication 7, **caractérisée en ce que** l'activation de l'élément moteur est réalisée par la fermeture du couvercle de l'enceinte au moyen d'un contacteur (39).

9. Enceinte selon l'une des revendications 6 à 8, **caractérisée en ce qu'**elle comporte un pupitre (40) de commande comportant un témoin (41) de fonctionnement pour chaque émetteur (30-33) de rayonnement et un témoin de visualisation de la position du volet (35) par rapport à la fenêtre (34).

## Patentansprüche

1. Dekontaminierungszelle für medizinische Instrumente, die durch einen Boden (2), zumindest eine Seitenwand (3, 4, 5, 6) und eine obere Abdeckung (7) begrenzt ist, wobei jedes Instrument (14) einen aktiven Bereich und einen Verbindungsbereich in Form eines Kabels (15) hat, **dadurch gekennzeichnet, daß** die Zelle einen Träger (10) umfaßt, der sich im Innern und teilweise oberhalb der Zelle (1) parallel zum Boden erstreckt und über dem Boden (2) auskragend angeordnet ist und mehrere Hängeelemente (12, 13) hat, von denen jedes dazu vorgesehen ist, mit einem an den aktiven Bereich des Instruments angrenzenden Kabelbereich (15) zusammenzuwirken.

2. Zelle nach Anspruch 1, **dadurch gekennzeichnet, daß** jedes Hängeelement einen Zapfen (12) umfaßt, der in eine obere Fläche des Trägers (10) eingelassen ist, sowie eine Hängeklammer (13), deren beiden Arme (18, 19) an einem mit einer Öffnung (21) versehenen Ende verbunden sind, um den Zapfen (12) durch Einstecken aufzunehmen, wobei die freien Enden der Arme der Klammer dazu geeignet sind, den an den aktiven Bereich des Instruments angrenzenden Bereich des Verbindungskabels (15) einzuklemmen.

3. Zelle nach Anspruch 2, **dadurch gekennzeichnet, daß** die Klammer ein Element (22) zum Bewegen der Arme (18, 19) umfaßt, um diese einander näher zu bringen oder voneinander zu beabstanden.

4. Zelle nach Anspruch 2 oder 3, **dadurch gekennzeichnet, daß** sie für das Verbindungskabel jedes mit der Seitenwand (3, 4, 5, 6) der Zelle formschlüssig verbundenen Instruments ein zweites Hängelement (16) hat, um das Verbindungskabel in einer im wesentlichen horizontalen Lage zwischen der Klammer und der Wand zu halten.

5. Zelle nach Anspruch 4, **dadurch gekennzeichnet, daß** das zweite Hängelement aus dem oberen Rand der Seitenwand (3) am Boden einer Einkerbung (16) der Seitenwand gebildet ist.

6. Zelle nach einem der vorhergehenden Ansprüche, **dadurch gekennzeichnet, daß** diese zumindest einen Sender (32, 33) hat, der germicide ultraviolette Strahlen aussendet und in einem Fenster (34) angeordnet ist, das in der Unterseite (2) der Zelle unterhalb des Trägers (10) liegt, und daß der Boden (2) mit einer Schutzabdeckung (35) für den Sender (32, 33) ausgestattet ist, die von einer ersten Stellung, in der sie das Fenster (34) bedeckt, in eine zweite Stellung, in der das Fenster abgedeckt ist, ausgerückt werden kann.

7. Zelle nach Anspruch 6, **dadurch gekennzeichnet, daß** die Abdeckung (35) an einem Element befestigt ist, das dazu vorgesehen ist, diese zwischen den beiden Stellungen hin- und herzubewegen und aus einem elastischen Element (36) gebildet ist zum Rückholen der Abdeckung (35) in die erste Stellung sowie aus einem Antriebselement (38), das im aktivierten Zustand gegenüber der Wirkung des elastischen Rückholelements (36) eine Kraft zum Bewegen der Abdeckung (35) in die zweite Stellung entwickelt.

8. Zelle nach Anspruch 7, **dadurch gekennzeichnet, daß** das Aktivieren des Antriebselements durch das Schließen der Abdeckung der Zelle mittels eines Kontaktgebers (39) bewirkt wird.

9. Zelle nach einem der Ansprüche 6 bis 8, **dadurch gekennzeichnet, daß** sie eine Schalttafel (40) umfaßt mit einer Funktionsanzeige (41) für jeden Strahlensender (30 bis 33) sowie einer Sichtanzeige zum Sichtbarmachen der Stellung der Abdeckung (35) relativ zu dem Fenster (34)

## Claims

1. A decontamination enclosure for medical instruments, the enclosure being defined by a bottom (2), at least one side wall (3, 4, 5, 6), and a top cover (7), each instrument (14) having an active portion and a connection portion in the form of a cable (15), the enclosure being **characterized in that** it comprises a bracket (10) extending inside the top portion of the enclosure (1) parallel to the bottom (2) and over the bottom (2), said bracket having a plurality of suspension members (12, 13) each being designed to co-operate with a portion of cable (15) adjacent to the active portion of the instrument.

2. An enclosure according to claim 1, **characterized in that** each suspension member comprises a finger (12) installed on a top surface of the bracket (10) and a suspension clamp (13) having two branches (18, 19) that are united at one end which is provided with an orifice (21) for being engaged on the above-mentioned finger (12), with the free ends of the branches of the clamp being shaped so as to be tightened onto the portion of the connection cable (15) that is adjacent to the active portion of the instrument.

3. An enclosure according to claim 2, **characterized in that** the clamp includes a member (22) for driving its branches (18, 19) so move them towards each other or away from each other.

4. An enclosure according to claim 2 or claim 3, **characterized in that** it includes a secondary suspension member (16) for the connection cable of each instrument and secured to the side wall of the enclosure (3, 4, 5, 6) so as to hold the cable in a substantially horizontal position between the clamp and the side wall.

5. An enclosure according to claim 4, **characterized in that** the secondary suspension member is constituted by the top edge of the side wall (3) at the bottom of a notch (16) therein.

6. An enclosure according to any preceding claim, the enclosure being **characterized in that** it comprises at least one emitter (32, 33) of ultraviolet germicidal radiation situated in a window (34) formed in the bottom wall (2) of the enclosure beneath the bracket (10), and **in that** the bottom (2) is fitted with a shutter (35) for protecting the emitter (32, 33), the shutter being retractable between a first position in which it covers the window (34) and a second position in which it leaves it uncovered.

7. An enclosure according to claim 6, **characterized in that** the shutter (35) is coupled to a member for driving it between the two above-mentioned positions, the member being formed by a resilient element (36) for returning the shutter (35) into its first position and by a drive element (38) which, in its active state, develops a force for displacing the shutter (35) towards its second position against the effect of the resilient return element (36).

8. An enclosure according to claim 7, **characterized in that** the drive element is activated by closing the cover of the enclosure, by means of a contact switch (39).

9. An enclosure according to any one of claims 6 to 8, **characterized in that** it includes a control panel (40) having an indicator (41) for indicating operation of each radiation emitter (30-33) and an indicator for displaying the position of the shutter (35) relative to the window (34).
